(19) Europäisches Patentamt
European Patent Office
Office européen des brevets

(11) EP 4 415 769 B1

(12) EUROPEAN PATENT SPECIFICATION

(45) Date of publication and mention of the grant of the patent:
**04.03.2026 Bulletin 2026/10**

(21) Application number: **22802550.8**

(22) Date of filing: **11.10.2022**

(51) International Patent Classification (IPC):
***A61L 15/24*** *(2006.01)* ***A61L 15/46*** *(2006.01)*
***D06M 13/265*** *(2006.01)* ***C08F 8/20*** *(2006.01)*
***C08F 8/32*** *(2006.01)* ***C08F 8/34*** *(2006.01)*
***D06M 13/342*** *(2006.01)* ***D06M 13/438*** *(2006.01)*
***D06M 14/10*** *(2006.01)*

(52) Cooperative Patent Classification (CPC):
(C-Sets available)
**A61L 15/46; A61L 15/24; C08F 8/20; C08F 8/32; C08F 8/34; D06M 13/265; D06M 13/342; D06M 13/438; D06M 14/10;** A61L 2300/202; A61L 2300/204; A61L 2300/216; D06M 2101/24; D06M 2101/26 (Cont.)

(86) International application number:
**PCT/EP2022/078253**

(87) International publication number:
**WO 2023/062008 (20.04.2023 Gazette 2023/16)**

# (54) FUNCTIONALIZED POLYMER PARTICLES OF FIBERS FOR ODOR CONTROL IN WOUND CARE

FUNKTIONALISIERTE POLYMERTEILCHEN AUS FASERN ZUR GERUCHSKONTROLLE IN DER WUNDVERSORGUNG

PARTICULES DE FIBRES POLYMÈRES FONCTIONNALISÉES POUR LA LUTTE CONTRE LES ODEURS DANS LE TRAITEMENT DES PLAIES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **15.10.2021 EP 21202928**

(43) Date of publication of application:
**21.08.2024 Bulletin 2024/34**

(73) Proprietor: **Mölnlycke Health Care AB**
**431 21 Mölndal (SE)**

(72) Inventors:
- **ELIAS, Milja**
  **44694 Skepplanda, Ale (SE)**
- **WELLNER, Eric**
  **41574 Göteborg (SE)**

(74) Representative: **Casalonga**
**Casalonga & Partners**
**Bayerstraße 71/73**
**80335 München (DE)**

(56) References cited:
**WO-A1-2016/130588**
**US-A1- 2005 008 608**
**US-A1- 2007 264 520**
**US-B2- 8 425 890**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 415 769 B1

(52) Cooperative Patent Classification (CPC): (Cont.)

C-Sets
**A61L 15/24, C08L 29/04;**
**A61L 15/24, C08L 33/08;**
**C08F 8/20, C08F 8/34, C08F 220/06;**
**C08F 8/32, C08F 220/06;**
**C08F 8/34, C08F 220/06**

## Description

Field of the Invention

The present invention relates to polymer particles or polymer fibers covalently bonded to N-chloroamines, N,N-dichloroamines, N-chloro sulfonamides or N,N-dichloro sulfonamides, for removing volatile organic compounds (VOCs) from a space above a wound, wherein the removal of said VOCs is primarily or predominantly by chemical reaction of the VOCs with the chloro-group as immobilized on the polymer.

In particular, the "active chlorine"-functionalized polymer particles or polymer fibers are part of a wound dressing and have the functionality to control, in particular reduce, odor emanating from wounds, without interacting with the wound, in particular without significantly or noticeably leaching into the wound. The dressings of the present invention can be advantageously used, in particular, in the treatment of chronic wounds or infected wounds.

In other aspects, the present invention relates to a method of controlling the odor above a wound space, said method including a step of providing the functionalized polymer particles or polymer fibers of the present invention.

Background of the Invention

As disclosed, for example, in the review article "A Comprehensive Review of Topical Odor-Controlling Treatment Options for Chronic Wounds" by A. Akhmetova et al., J Wound Ostomy Continence Nurs. (2016), 43(6), pages 598 to 609, various methods or products are available to manage wound odor. One of the more commonly used compositions for odor control over wounds is activated charcoal (which may be part of wound dressing). Charcoal is generally understood to absorb or "trap" VOCs due to the large active surface area of (activated) charcoal. No chemical reaction is believed to be involved in this physical adsorption mechanism.

In other approaches, silver or iodine are used as components in wound dressings, for example as coatings on a non-woven material or as impregnated in viscose rayon. Silver is believed to *indirectly* reduce odor by way of its antimicrobial properties. In that sense, silver should rather be seen as an antimicrobial agent (and as such may be part of the wound dressing of the present invention) than an odor reducing agent. Similarly, iodine is used to reduce the bacterial burden in the wound bed and therefore also reduces odor by way of disrupting bacterial wall membranes.

In an alternative approach, metronidazole (which is commonly used as a topically applied antibiotic) is used for the management of wound odor because of its ability to reduce odor producing anaerobic pathogens in selected wounds.

Natural compounds are also known as means to control wound malodor, for example honey or essential oils.

None of these approaches (silver, iodine, antibiotics, "natural" antibacterial products) are based on a mechanism of chemically reacting with the VOCs produced by bacteria/microbes/germs.

While all the known approaches as described above have their advantages, they are also associated with certain limitations. For example, the problem with wound control measures that involve physical adsorption, in particular charcoal, is the potential of saturation as the VOCs are not converted into non-odorous substances but are *"stored"* until capacity is reached. Furthermore, indirect methods that reduce or kill bacteria are not suitable for all wound types and only deal with odor due to bacterial infection. Such methods may not be suitable for all wounds, in particular not for chronic wounds.

Furthermore, many of these known approaches require that the (antimicrobial) agent penetrates into the wound in order to be effective, i.e. the agent is indeed an active ingredient that requires full clinical studies and certification.

Other sources of volatile organic compounds are metabolites produced by humans and secreted via the skin. Also, necrotic tissue as commonly present in chronic wounds may release VOCs.

A known approach to address odors in hygiene articles (e.g. in diapers) is the use of sulfonamides. As an example, US 8,425,890 describes halo active aromatic sulfonamides for use in controlling odor in bodily fluids in personal hygiene articles such as diapers and sanitary napkins. According to the prior art, the sulfonamides, for example the halo active aromatic sulfonamides according to US 8,425,890 are used as *such,* i.e. are used as chemical compounds that are added (e.g. coated onto) the overall product. For example, in US 8,425, 890, wood fluff is treated with an aqueous solution of the sulfonamide compounds.

Simply spraying chemical compounds onto a substrate (and then enclosing the substrate inside the article) may be a suitable approach for hygiene articles that are meant to take up bodily fluids secreted from the human body. However, the situation is different for wound dressings since wound dressings come in immediate contact with wound beds typically comprising open or compromised blood vessels, which means that the entry of chemically active compounds into the wound is generally not desirable or may even be detrimental to wound healing. Therefore, wound dressings that comprise active compounds that may leach out of the dressing are not desirable and the concept disclosed in US 8,425, 890 cannot be readily transferred to wound dressings.

Summary of the Invention

Overall, in view of the above, one object of the present invention is to provide means for controlling odor, in particular odor above wounds, wherein said means avoid or mitigate any or all of the disadvantages mentioned above.

In particular, an object of the present invention is to provide odor control means for wounds, in particular for chronic wounds, that do not expose the wound to direct contact with reactive chemical compounds and that permanently remove volatile organic compounds irrespective of their source.

This problem and others is/are solved, in a first aspect, by polymer particles or polymer fibers that are covalently bonded to N-chloroamines, or N,N-dichloroamines or covalently bonded to N-chloro or N,N-dichloro sulfonamides, wherein said functionalized polymer fibers or particles are used for removing volatile organic compounds from a space above a wound, wherein the moiety as bonded to the polymer particles or polymer fibers is of a structure selected from the following:

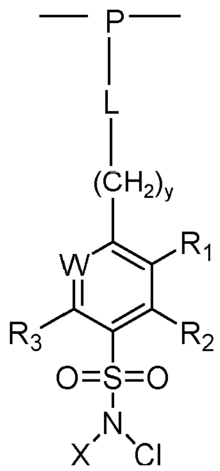

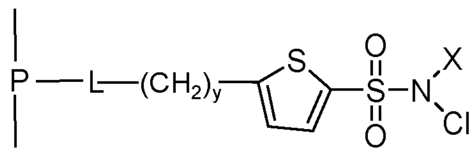

$P\text{-}L\text{-}(CH_2)_y\text{-}SO_2\text{-}NXCl$ or $P\text{-}L\text{-}(CH_2)_y\text{-}NXCl$,

wherein:

P- is a polymer;
L is a linker;
y is an integer greater than zero;
W is N or -CH;
$R_1$, $R_2$ and $R_3$ are independently selected from -H-, -alkyl, or -halogen;
X is Na, H or Cl.

Without wishing to be bound by theory, the chlorine group of the moiety that is covalently bonded to the polymer is believed to remove VOCs primarily or predominantly by chemical reaction, i.e. the VOCs are cleaved into non (or less) odorous reaction products.

In embodiments, one of the following, preferably all of the following applies:

$L\text{-}(CH_2)_y$ is $\text{-}NH\text{-}(CH_2)_y$; and/or

y is from 1 to 20, preferably from 1 to 12 or from1 to 6 and/or

$R_1$ is selected from -H-, $\text{-}CH_3\text{-}$, or -Cl-; and/or

halogen is F, Cl or Br and/or:

$R_2$ and $R_3$ are -H-.

In embodiments particularly relating to the claimed N-chloro sulfonamides or N,N-dichloro sulfonamides, y is from 1 to 3

In embodiments particularly relating to the claimed N-chloroamines or N,N-dichloroamines, y is from 2 to 12

In embodiments, the polymer of the polymer particles or polymer fibers is based on polyacrylic acid, partially hydrolyzed acetyl-poly(vinylalcohol) or poly(vinyl alcohol), optionally a cross-linked polymer network of polyacrylic acid, partially hydrolyzed acetyl-poly(vinylalcohol) or poly(vinyl alcohol), or any copolymer or mixture thereof, wherein polyacrylic acids are preferred.

In embodiments, at least 0.1% of the alcohol or carboxylic acid groups originally present in the polymer are functionalized with a N-chloro or N,N--dichloro unit, preferably at least 0.5%.

In embodiments, the fraction of modified of polymer, i.e. degree of functionalization of the alcohol or carboxylic acid groups originally present in the polymer with a N-chloro or N,N- -dichloro unit, is from 0.1% to 80%, preferably from 0.5% to 50%.

In embodiments, the amount of active chlorine as determined by the method provided in the experimental section below is from 0.1% to 20% (w/w), optionally from 0.3% to 16% (w/w).

In embodiments, the polymer particles are superabsorbent polymer particles (SAPs).

In accordance with the present invention and in accordance with the IUPAC definition provided in Pure Appl. Chem., Vol. 76, No. 4, pp. 889-906, 2004, a superabsorbent polymer is a polymer that can absorb and retain extremely large amounts of liquid relative to its own mass, preferably 10 times its weight or 100 times its weight.

In embodiments, the polymer fibers are superabsorbent fibers (SAFs).

In embodiments the median $D^{50}$ particle diameter of the polymer particles is from 80 μm to 600 μm, optionally from 150 μm to 400 μm; or wherein the average diameter of the fibers is from 1 μm to 300 μm, optionally from 5 μm to 100 μm.

In a second aspect, the above problem is / the above problems are solved by a wound dressing comprising the polymer particles or polymer fiber as described herein.

In embodiments, the fibers according to the present invention are preferentially incorporated in an airlaid fiber structure while the particles according to the present invention are preferentially incorporated in a foam.

In embodiments, at least 5% by weight, relative to the overall weight, optionally at least 10% by weight of the wound dressing is provided by the polymer particles or polymer fiber as described herein.

In embodiments, the polymer particles or polymer fibers as described herein have a grammage of at least 15 $g/m^2$, optionally at least 30 $g/m^2$ of the overall wound dressing.

In embodiments, the wound dressing furthermore comprises at least one of the following: (a) a backing layer; (b) at least one absorbent layer; (c) a wound contact layer.

In embodiments, said wound contact layer comprises or consists of a silicone gel.

In embodiments, the polymer particles or polymer fibers according to the present invention are provided as part of the absorbent layer.

In embodiments, the absorbent layer comprises superabsorbent particles and/or superabsorbent fibers *not* functionalized with N-chloro or N,N-dichloro groups, i.e. SAPs and/or SAFs, the primary function of which is to absorb wound exudate.

In embodiments, the wound dressing comprises superabsorbent particles and/or superabsorbent fibers *not* functionalized with N-chloro or N,N-dichloro sulfonamides. Wherein, in addition, polymer particles or polymer fibers functionalized with N-chloro or N,N-dichloro groups are provided *separately* from said superabsorbent particles or superabsorbent fibers not functionalized with N-chloro or N,N-dichloro groups.

In embodiments, the polymer particles or polymer fibers functionalized with N-chloro or N,N-dichloro groups are provided in a layer farther away from the area of the wound dressing that is intended to be put in contact with the wound (wound contact layer) relative to the layer that comprises the superabsorbent particles or superabsorbent fibers not functionalized with N-chloro or N,N-dichloro groups. This spatial separation ensures that those SAPs or SAFs that have the primary functionality of absorbing wound exudate are indeed close to the wound, while those SAPs and SAFs that are functionalized to cleave VOCs are removed away from the wound.

In a third aspect, the above-mentioned problem(s) is/are solved by a method of controlling odor above a wound space, said method comprising at least the following steps:

(a) providing polymer particles or polymer fibers as described above and herein in a wound dressing;
(b) bringing at least a fraction of said polymer particles or polymer fibers into contact with at least one volatile organic compound that is exuded from a wound and/or from the human skin in the vicinity of a wound;
(c) chemically reacting said volatile organic compound with N-chloro or N,N-dichloro groups as covalently bonded to said polymer particles or polymer fibers thus at least partially reducing odor emanating from a wound or from the area around a wound.

In embodiments, said at least one volatile organic compound is selected from heteroarylic, arylic compounds, sulfides, di-sulfides, trisulfides, ketones, alcohols, aldehydes, amines or carboxylic acids and esters.

In embodiments, said at least one volatile organic compound is selected from electron rich heteroarylic and arylic compounds.

In embodiments, said at least one volatile organic compound is selected from indole, 3-methyl indole, pyrimidine, acetophenone, isovaleric acid, 2,5-dimethylpyrazine, pyrrole, p-cresol, 2-aminoacetophenone, 6-methyl-5-heptene-2-one, cadaverine.

In embodiments, the wound is selected from chronic wounds (in contrast to "acute" wounds) and/or infected wounds.

**Brief Description of the Figures**

**Figure 1** shows a synthesis scheme for functionalizing polymer particles or fibers with mono- or dichloro sulfonamide groups (1) and with mono or dichloro-amine groups (2). The synthesis is described in more detail below in the Experimental Section.
In this figure, EDC-HCl is N-(3-Dimethylaminopropyl)-N'-ethylcarbodiimide hydrochloride, NHS is N-Hydroxy-succinimide, MES is 4-Morpholineethanesulfonic acid monohydrate, HCl is Hydrochloric acid, $NaHCO_3$ is Sodium bicarbonate, NaOCl is sodium hypochlorite and HAc is Acetic acid.

**Figure 2** shows SEM images of A) non-functionalized superabsorbent fibers (SAFs) B) N-chloro-sulfonamide modified SAF and C) N,N-dichloro-sulfonamide modified SAF.
As can be taken from the scanning electron microscopy pictures, the diameter of unmodified SAF is around 30μm whereas the diameter of N-chloro-sulfonamide modified SAF is around 40μm and that of N,N-dichloro-sulfonamide modified SAF around 50μm.

**Figure 3** shows that superabsorbent particles (here: polyacrylic acid, these particles are also referred to as "PAA") and superabsorbent fibers SAF (also based on polyacrylic acid) as *such,* i.e. *without being modified* in accordance with the present invention essentially do not affect (reduce) the amount of volatile organic compounds. In these Figures and in the following Figures the VOCs are: Indole, 3-Methyl indole (3MeInd), Pyrimidine (Pyrim), Acetophenone (APh), Isovaleric acid (Isoval), 2,5-Dimethylpyrazine (2,5-Dimepy), Pyrrole, p-Cresol, 2-aminoacetophenone (2-AAph), 6-Methyl-5-heptene-2-one (6M-hept-one) and Cadaverine (CAD). The one exception to the general observation that the unmodified polymers do not reduce the amount of VOCs is that superabsorbent fibers as such indeed do reduce (or even remove) cadaverine (see lower panel of Figure 3).

**Figures 4 to 7** show how functionalization of SAPs and SAFs with mono- or dichloro sulfonamide groups or with mono or dichloro-amine groups leads to a significant to essentially complete removal of a large range of VOCs.

Figure 4, upper panel, shows the effect of functionalized SAF polymer 2 as described in the experimental section below on selected VOCs while Figure 4, lower panel, shows the effect of functionalized SAP Polymer 1 as described in the experimental section below on selected VOCs

Figure 5, upper panel, shows the effect of different amounts of functionalized SAP Polymer 2 as described in the experimental section below on selected VOCs while Figure 5, lower panel, shows the effect of functionalized SAF Polymer 3 as described in the experimental section below on selected VOCs

Figure 6, upper panel, shows the effect of functionalized SAF polymer 5 as described in the experimental section below on selected VOCs while Figure 6, lower panel, shows the effect of functionalized SAF Polymer 4 as described in the experimental section below on selected VOCs

Figure 7, upper panel, shows the effect of functionalized SAF polymer 6 as described in the experimental section below on selected VOCs while Figure 7, lower panel, shows the effect of functionalized SAF Polymer 7 as described in the experimental section below on selected VOCs

**Figures 8 and 9** show tables providing values for the % reduction of VOCs for functionalized polymers as described in the Experimental Section below.

## Detailed Description of Embodiments of the Invention

One advantage of the present invention is that the volatile organic compounds are not simply absorbed but that an actual chemical reaction takes place that converts the odorous VOCs into non-odorous or otherwise harmless smaller molecules. Since a chemical reaction takes place, there is, in principle, no limitation to the amount of VOCs that can be removed from a wound space or the vicinity of a wound space by the functionalized polymer particles or fibers as described herein.

Also, importantly, due to immobilization of the chemically reactive moiety with the N-chloro or the N,N dichloro group to a polymer backbone, release of a water soluble active component into the wound is avoided or at least substantially avoided.

This puts the present invention in contrast to existing odor control measures in hygiene articles, wherein sulfonamides are not truly immobilized or not at all immobilized, for example merely sprayed or coated onto a substrate and thus may be easily released into a fluid system and thus may leach into the wound, which is generally undesirable (see "Background" section)

A further advantage of the present invention is that polymer particles or polymer fibers that are already commonly used in wound dressings, for example superabsorbent polymers or superabsorbent fibers can be functionalized to carry the herein disclosed N-chloro or the N,N dichloro group

In embodiments, superabsorbent polymer particles or superabsorbent fibers are partially functionalized with the N-chloro or the N,N dichloro group as disclosed herein thus leading to superabsorbent particles or superabsorbent fibers that are still superabsorbent but that also have VOCs cleaving functionality.

In accordance with the present invention "partially functionalized" means that 5% to 75% of all available OH-groups of a particle or fiber are functionalized, preferably 15% to 60%.

In other embodiments, polymer fibers or polymer particles are fully or essentially fully functionalized with N-chloro or the N,N dichloro groups as disclosed herein and such fully or essentially fully functionalized superabsorbent fibers or superabsorbent particles are used *together* with the same or different superabsorbent polymer particles or superabsorbent polymer fibers that are not functionalized or are only partially functionalized and thus primarily function as superabsorbent materials.

In other embodiments, several layers are provided in a wound dressing, wherein the layer closer to the wound space comprises superabsorbent particles or superabsorbent fibers that are not functionalized or only partially functionalized with N-chloro or N,N dichloro groups while a second, separate layer of superabsorbent particles or superabsorbent fibers is provided farther away from the wound space, which then comprises fully essentially fully functionalized superabsorbent polymers or superabsorbent fibers having the primary functionality to cleave VOCs.

A further advantage of the present invention is that due to the mechanism of chemically reacting with VOCs, i.e. *directly* removing the VOCs, the mechanism that leads to VOCs or the origin of the VOCs is irrelevant and wounds can be treated in which the origin of the VOCs is not primarily of bacterial origin.

An additional benefit of the sulfonamide functionalized polymer particles or polymer fibers is that in use, i.e. in contact with wound exudate, the claimed and disclosed N-chloro or N,N dichloro group are capable of releasing hypochlorite which is known to have antimicrobial properties and can thus also contribute to the lowering of the bioburden in a dressing and/or in the wound.

## Examples (Experimental)

**1.) Synthesis** (see Figure 1)

Reagents:

Polyacrylic acid (PAA) - Mol. Wt. - 1,250,000 Da - Sigma Aldrich

SAF - Super absorbent fiber - Technical Absorbents

AEBSA - 4-(2-Aminoethyl)benzenesulfonamide, 99% - Thermo Fisher Scientific

MES - 4-Morpholineethanesulfonic acid monohydrate - Thermo Scientific™ BupH MES Buffered Saline Pack

NaOCl - sodium hypochlorite (6-14% active chlorine) - Honeywell Fluka

HAc - glacial acetic acid - Sigma Aldrich

N-Boc-ethylenediamine - Sigma Aldrich

N-Boc-1,6-hexanediamine - Sigma Aldrich

Synthesis of polyacrylic acid polymer particles functionalized with N-chloro/N,N-dichloro sulfonamide

Step 1 - EDC coupling

1g PAA was added to 65 mL MES buffer (0.05M, pH=6.0) in a 250 mL RB flask. Then 0.760g EDC-HCl and 0.920g NHS dissolved in 15 mL MES buffer (0.05M, pH=6.0) was added. This was stirred at RT for 30 minutes. Then 1.2g AEBSA dissolved in 20 mL phosphate buffer solution (0.1M, pH=7.0) was added and the reaction continued at RT for 4h. After 4h, the sulfonamide bound polymer was washed with deionized water to remove all the unreacted reagents and impurities. The formed product was then dried at RT.

Step 2 - Chlorination

**Polymer 1** - 1g of the sulfonamide bound polymer was taken in a round bottom flask and added deionized water to swell the polymer. Then 16mL of NaOCl solution and 1mL HAc was added. The reaction was continued in an ice bath for 4h. The formed product was washed with large amount of deionised water till the washings did not show presence of chlorine (Absence of blue color with KI and starch solution).

Synthesis of polymer fibers functionalized with N-chloro/N,N-dichloro sulfonamides

Step 1- EDC coupling

1g SAF was added to a 250 mL RB flask followed by 20 mL of MES buffer. 0.76g EDC and 0.92g NHS was dissolved in 8 mL of MES buffer and added to SAF in MES buffer. After stirring for 30 minutes, 1.2g AEBSA dissolved in 20 mL of phosphate buffer (pH= 7.0) was added. Stirred for 4h. The product was washed several times with deionised water and dried at room temperature.

Step 2 - Chlorination

**Polymer 2** - 1g of sulfonamide bound superabsorbent fibers (SAF) was added to 250mL RB flask. Approx.120mL deionised water was added to swell the fiber. Then 16mL NaOCl and 1mL gl. acetic acid was added and the reaction continued for 4h in an ice bath. The formed product was washed with large amount of deionised water till the washings did not show presence of chlorine (Absence of blue color with KI and starch solution). Dried at room temperature.

**Polymer 3** - 1g of sulfonamide bound SAF was added to 250mL RB flask. Approx. 120mL deionised water was added to swell the fiber. Then 16mL NaOCl was added and the reaction continued for 4h in an ice bath. The formed product was washed with large amount of deionised water till the washings did not show presence of chlorine (Absence of blue color with KI and starch solution). Dried at room temperature.

Synthesis of polymer fibers functionalized with N-chloro/N,N-dichloroamines

Step 1- EDC coupling was carried out as mentioned above for SAF with N-Boc protected diamines instead of AEBSA.

Step 2- Deprotection:- 0.25 g of fibers modified with N-Boc protected amine was added to 10 mL 4MHCl and stirred at room temperature for 3h. This was filtered, added deionised water and neutralised with sodium bicarbonate. The formed amine modified fibers were filtered, washed with deionized water and dried at RT

Step 3 - Chlorination

**Polymer 4** - Same procedure was used as for polymer 2 to get N,N-dichloro- ethylene diamine modified SAF.

**Polymer 5** - Same procedure was used as for polymer 3 to get N-chloro-ethylene diamine modified SAF.

**Polymer 6** - Same procedure as for polymer 2 to get N,N-dichloro-hexamethylene diamine modified SAF.

**Polymer 7** - Same procedure as for polymer 3 to get N-chloro-hexamethylene diamine modified SAF.

**2.) Determination of amount of active chlorine in the modified polymers-Iodometric titration**

0.5g of KI was added to 10 mL deionized water. 50 mg of the modified polymer was added followed by 3 drops of 1% starch solution as indicator. A blue color formed which disappears at end point upon titration with 0.1 N (for N,N-dichloro compounds) or 0.01 N (for N-chloro compounds) sodium thiosulphate solution. Titration was done in triplicates for all samples.

The amount of active chlorine was calculated using the following equation:

$$Cl^{+} (\%) = \frac{35.45 X N X V}{2 X W} X\ 100$$

wherein N is the equivalent concentration (N) and V is the volume (L) of the sodium thiosulphate solution used for titration, W is the weight of the modified polymer (g).

The following amounts of active chlorine were determined using this titration method:

Polymer 1 - Polyacrylic acid modified with N,N-dichlorosulfonamide ~4%
Polymer 2 - SAF modified with N,N-dichlorosulfonamide - ~4% and ~14%
Polymer 3 - SAF modified with N-chlorosulfonamide - ~ 2%
Polymer 4 - SAF modified with N,N-dichloro-ethylene diamine ~8%
Polymer 5 - SAF modified with N-chloro-ethylene diamine - ~ 0.3 - ~0.6%
Polymer 6 - SAF modified with N,N-dichloro-diaminohexane - ~ 8%
Polymer 7 - SAF modified with N-chloro-diaminohexane - ~ 1%

**3.) Testing reduction of VOC concentration with modified polymers**

VOCs emitted by bacteria commonly found in wound (P. aeruginosa, S. aureus, E. coli) were selected from literature. These were divided into two mixtures for ease of calibration, because of co-eluting peaks, to get good peak separation.

Mix 1:- Indole, 3-Methyl indole (3MeInd), Pyrimidine (Pyrim), Acetophenone (APh), Isovaleric acid (Isoval), 2,5-Dimethylpyrazine (2,5-Dimepy)

Mix 2:- Pyrrole, p-Cresol, 2-aminoacetophenone (2-AAph), 6-Methyl-5-heptene-2-one (6M-hept-one), Cadaverine (CAD).

Method:

- VOCs -A concentration in the validated range was chosen and the solutions were prepared as Mix 1 and Mix 2 in simulated wound fluid (SWF).
- Polymer material (polymer 1 to 7, 100 mg) was added to 2.5 mL each of Mix1 and Mix 2 solution in SWF. For polymer 2 the amount was varied as specified in Figure 8.
- Vortexed for one minute.
- Incubated with shaking at 35 deg C for 24h.
- Supernatant analysed by HPLC-MS.

The concentration of VOCs in SWF after incubation for 24h was measured and from that the percentage reduction after adding the materials was calculated.

### 4.) Results

As shown in Figure 3, the non-modified (non-functionalized) polymer particles or fibers do not affect the VOC concentration (with the exception of SAFs reducing cadaverine).

Figures 4 to 7 as well as the tables reproduced in Figure 8 and Figure 9 show that, depending on the specific modification (Polymers 1 to 7 as described above) essentially all of the exemplarily chosen VOCs can be removed by way of chemical reaction. Figure 5 shows that at least VOCs can already be removed by a comparatively low amount of functionalized polymer.

Figures 4 to 9 provide evidence that the inventive concept works well for both polymer particles and fibers and that functionalization with chloroamines and with chlorosulfonamides is effective in reducing VOCs.

## Claims

**1.** Polymer particles or polymer fibers covalently bonded to N-chloroamines or N,N-dichloroamines or covalently bonded to N-chloro or N,N-dichloro sulfonamides, for removing volatile organic compounds from a space above a wound, wherein the moiety as bonded to the polymer particles or polymer fibers is of a structure selected from the following:

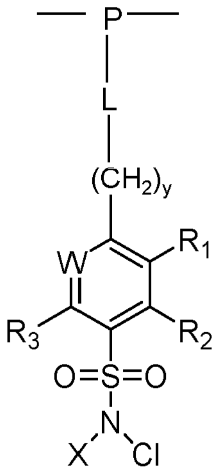
,
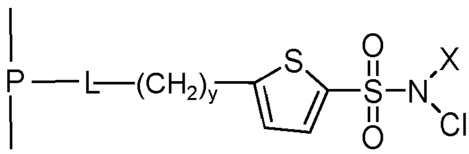
,

$$P\text{-}L\text{-}(CH_2)_y\text{-}SO_2\text{-}NXCl \text{ or } P\text{-}L\text{-}(CH_2)_y\text{-}NXCl,$$

wherein:

P- is a polymer;
L is a linker;
y is an integer greater than zero
W is N or -CH;
$R_1$ , $R_2$ and $R_3$ are independently selected from -H-, -alkyl, or -halogen;
X is Na, H or Cl.

**2.** Polymer particles or polymer fibers of claim 1, wherein

$L\text{-}(CH_2)_y$ is $\text{-}NH\text{-}(CH_2)_y$; and/or
y is from 1 to 20, preferably from 1 to 12 or from 1 to 6 and/or
$R_1$ is selected from -H-, $\text{-}CH_3\text{-}$, or -Cl-; and/or
halogen is F, Cl or Br and/or:
$R_2$ and $R_3$ are -H-.

**3.** Polymer particles or polymer fibers of claim 1 or claim 2, wherein the polymer is based on polyacrylic acid, partially hydrolyzed acetyl-poly(vinylalcohol) or poly(vinyl alcohol), optionally a cross-linked polymer network of polyacrylic acid, partially hydrolyzed acetyl-poly(vinylalcohol) or poly(vinyl alcohol), or any copolymer or mixture thereof,

preferably wherein the polymer is based on polyacrylic acid.

4. Polymer particles or polymer fibers according to any one of the preceding claims, wherein at least 0.1% of the alcohol or carboxylic acid groups originally present in the polymer are functionalized with a N-chloro or N,N--dichloro unit, preferably at least 0.5%.

5. Polymer particles or polymer fibers according to any one of the preceding claims, wherein the amount of active chlorine as determined by the method provided in the description is from 0.1% to 20% (w/w), optionally from 0.3% to 15%.

6. Polymer particles or polymer fibers according to any one of the preceding claims, wherein the median D50 particle diameter of the polymer particles is from 80 μm to 600 μm, optionally from 150 μm to 400 μm; or wherein the average diameter of the fibers is from 1 μm to 300 μm, optionally from 5 μm to 100 μm.

7. Wound dressing, comprising the polymer particles or polymer fibers of any one of the preceding claims.

8. Wound dressing according to claim 7, comprising at least 5% by weight of said polymer particles or polymer fibers, relative to the overall weight of the dressing, optionally at least 10% by weight and/or wherein the polymer particles or polymer fibers of any one of the preceding claims have a grammage of at least 15 $g/m^2$, optionally at least 30 $g/m^2$ of the overall wound dressing.

9. Wound dressing according to claim 7, furthermore comprising at least one of the following:

(a) a backing layer;
(b) at least one absorbent layer;
(c) a wound contact layer, said wound contact layer optionally comprising a silicone gel.

10. Wound dressing according to claim 8 or claim 9, wherein the polymer particles or polymer fibers are provided as part of the absorbent layer, optionally wherein the absorbent layer comprises superabsorbent particles or superabsorbent fibers not functionalized with N-chloro or N,N-dichloro groups.

11. Wound dressing according to any one of claims 7 to 10, wherein the wound dressing also comprises superabsorbent particles or superabsorbent fibers not functionalized with N-chloro or N,N-dichloro sulfonamides. wherein the polymer particles or polymer fibers functionalized with N-chloro or N,N-dichloro sulfonamides are provided separately from the superabsorbent particles or superabsorbent fibers not functionalized with N-chloro or N,N-dichloro sulfonamides, in particular wherein the polymer particles or polymer fibers functionalized with N-chloro or N,N-dichloro sulfonamides are provided in a layer farther away from the area of the wound dressing that is intended to be put in contact with the wound than a layer that comprises the superabsorbent particles or superabsorbent fibers not functionalized with N-chloro or N,N-dichloro groups.

12. Method of controlling odor above a wound space, said method comprising at least the following steps:

(a) providing polymer particles or polymer fibers according to any one of claims 1 to 6 in a wound dressing;
(b) bringing at least a fraction of said polymer particles or polymer fibers into contact with at least one volatile organic compound that is exuded from a wound and/or from the human skin in the vicinity of a wound;
(c) chemically reacting said volatile organic compound with the N-chloro or N,N-dichloro group as covalently bonded to said polymer particles or polymer fibers thus at least partially reducing odor emanating from a wound or from the area around a wound.

13. Method according to claim 12, wherein said at least one volatile organic compound is selected from heteroarylic, arylic compounds, sulfides, di-sulfides, trisulfides, ketones, alcohols, aldehydes, amines or carboxylic acids and esters.

14. Method according to claim 12, wherein said at least one volatile organic compound is selected from indole, 3-methyl indole, pyrimidine, acetophenone, isovaleric acid, 2,5-dimethylpyrazine, pyrrole, p-cresol, 2-aminoacetophenone, 6-methyl-5-heptene-2-one, cadaverine.

15. Wound dressing or method according to any one of claims 1 to 14, wherein the wound is selected from chronic wounds and/or infected wounds.

**Patentansprüche**

1. Polymerpartikel oder Polymerfasern, die kovalent an N-Chloramine oder N,N-Dichloramine oder kovalent an N-Chlor- oder N,N-Dichlorsulfonamide gebunden sind, zum Entfernen flüchtiger organischer Verbindungen aus einem Raum über einer Wunde, wobei die an die Polymerpartikel oder Polymerfasern gebundene Einheit eine Struktur aufweist, die aus den folgenden ausgewählt worden ist:

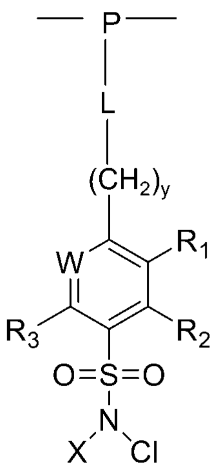
,
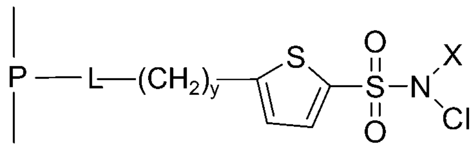
,

$$P\text{-}L\text{-}(CH_2)_y\text{-}SO_2\text{-}NXCl \text{ oder } P\text{-}L\text{-}(CH_2)_y\text{-}NXCl,$$

wobei

P- ein Polymer ist,
L ein Linker ist,
y eine ganze Zahl größer als Null ist,
W N oder -CH ist,
$R_1$, $R_2$ und $R_3$ unabhängig voneinander aus -H-, -Alkyl oder -Halogen ausgewählt worden sind,
X Na, H oder Cl ist.

2. Polymerpartikel oder Polymerfasern nach Anspruch 1, wobei

L-$(CH_2)_y$ -NH-$(CH_2)_y$ ist, und/oder
y 1 bis 20, bevorzugt 1 bis 12 oder 1 bis 6 ist und/oder
$R_1$ aus -H-, -$CH_3$- oder -Cl- ausgewählt worden ist, und/oder
Halogen F, Cl oder Br ist und/oder:
$R_2$ und $R_3$ -H- sind.

3. Polymerpartikel oder Polymerfasern nach Anspruch 1 oder Anspruch 2, wobei das Polymer auf Polyacrylsäure, teilweise hydrolysiertem Acetyl-Poly(vinylalkohol) oder Poly(vinylalkohol), basiert, optional einem vernetzten Polymernetzwerk aus Polyacrylsäure, teilweise hydrolysiertem Acetyl-Poly(vinylalkohol) oder Poly(vinylalkohol) oder einem beliebigen Copolymer oder einer Mischung davon basiert, wobei das Polymer bevorzugt auf Polyacrylsäure basiert.

4. Polymerpartikel oder Polymerfasern nach einem der vorstehenden Ansprüche, wobei mindestens 0,1 %, bevorzugt mindestens 0,5 %, der ursprünglich in dem Polymer vorhandenen Alkohol- oder Carbonsäuregruppen mit einer N-Chlor- oder einer N,N-Dichlor-Einheit funktionalisiert sind.

5. Polymerpartikel oder Polymerfasern nach einem der vorstehenden Ansprüche, wobei die Menge an aktivem Chlor, bestimmt nach dem in der Beschreibung angegebenen Verfahren, 0,1 % bis 20 % (Gew./Gew.), optional 0,3 % bis 15 % beträgt.

6. Polymerpartikel oder Polymerfasern nach einem der vorstehenden Ansprüche, wobei der mittlere D50-Partikeldurchmesser der Polymerpartikel 80 μm bis 600 μm, optional 150 μm bis 400 μm beträgt, oder wobei der durchschnittliche Durchmesser der Fasern 1 μm bis 300 μm, optional 5 μm bis 100 μm beträgt.

7. Wundverband, welcher die Polymerpartikel oder Polymerfasern nach einem der vorstehenden Ansprüche aufweist.

8. Wundverband nach Anspruch 7, welcher mindestens 5 Gewichtsprozent der Polymerpartikel oder Polymerfasern, bezogen auf das Gesamtgewicht des Verbands, optional mindestens 10 Gewichtsprozent aufweist, und/oder wobei die Polymerpartikel oder Polymerfasern aus einem der vorstehenden Ansprüche ein Flächengewicht von mindestens 15 $g/m^2$, optional mindestens 30 $g/m^2$ des gesamten Wundverbands aufweisen.

9. Wundverband nach Anspruch 7, welcher außerdem mindestens eines der folgenden Elemente aufweist:

(a) eine Trägerschicht ("backing layer"),
(b) mindestens eine absorbierende Schicht,
(c) eine Wundkontaktschicht, wobei die Wundkontaktschicht optional ein Silikongel aufweist.

10. Wundverband nach Anspruch 8 oder Anspruch 9, wobei die Polymerpartikel oder Polymerfasern als Teil der absorbierenden Schicht vorgesehen sind, wobei die absorbierende Schicht optional superabsorbierende Partikel oder superabsorbierende Fasern aufweist, die nicht mit N-Chlor- oder N,N-Dichlor-Gruppen funktionalisiert worden sind.

11. Wundverband nach einem der Ansprüche 7 bis 10, wobei der Wundverband auch superabsorbierende Partikel oder superabsorbierende Fasern aufweist, die nicht mit N-Chlor- oder N,N-Dichlor-Sulfonamiden funktionalisiert worden sind, wobei die Polymerpartikel oder Polymerfasern, die mit N-Chlor- oder N,N-Dichlorsulfonamiden funktionalisiert worden sind, getrennt von den superabsorbierenden Partikeln oder superabsorbierenden Fasern, die nicht mit N-Chlor- oder N,N-Dichlorsulfonamiden funktionalisiert worden sind, bereitgestellt sind, insbesondere wobei die Polymerpartikel oder Polymerfasern, die mit N-Chlor- oder N, N-Dichlorsulfonamiden funktionalisiert worden sind, in einer Schicht bereitgestellt werden, die weiter entfernt ist von dem Bereich des Wundverbands, der mit der Wunde in Kontakt gebracht werden soll, als eine Schicht, welche die superabsorbierende Partikel oder superabsorbierende Fasern aufweist, die nicht mit N-Chlor- oder N,N-Dichlor-Gruppen funktionalisiert worden sind.

12. Verfahren zur Geruchskontrolle über einem Wundraum, wobei das Verfahren mindestens die folgenden Schritte umfasst:

(a) Bereitstellen von Polymerpartikeln oder Polymerfasern gemäß einem der Ansprüche 1 bis 6 in einem Wundverband;
(b) In-Kontakt-Bringen mindestens eines Teils der Polymerpartikel oder Polymerfasern mit mindestens einer flüchtigen organischen Verbindung, die aus einer Wunde und/oder aus der menschlichen Haut in der Nähe einer Wunde austritt;
(c) chemisches Reagieren der flüchtigen organischen Verbindung mit der N-Chlor- oder N,N-Dichlor-Gruppe, die kovalent an die Polymerpartikel oder Polymerfasern gebunden ist, wodurch der von einer Wunde oder dem Bereich um eine Wunde herum ausgehende Geruch zumindest teilweise verringert wird.

13. Verfahren nach Anspruch 12, wobei die mindestens eine flüchtige organische Verbindung aus Heteroaryl-, Aryl-Verbindungen, Sulfiden, Disulfiden, Trisulfiden, Ketonen, Alkoholen, Aldehyden, Aminen oder Carbonsäuren und Estern ausgewählt worden ist.

14. Verfahren nach Anspruch 12, wobei die mindestens eine flüchtige organische Verbindung aus Indol, 3-Methylindol, Pyrimidin, Acetophenon, Isovaleriansäure, 2,5-Dimethylpyrazin, Pyrrol, p-Kresol, 2-Aminoacetophenon, 6-Methyl-5-hepten-2-on und Cadaverin ausgewählt worden ist.

15. Wundverband oder Verfahren nach einem der Ansprüche 1 bis 14, wobei die Wunde aus chronischen Wunden und/oder infizierten Wunden ausgewählt worden ist.

**Revendications**

**1.** Particules de polymère ou fibres de polymère liées de manière covalente à des N-chloroamines ou N,N-dichloroamines ou liées de manière covalente à des N-chloro sulfonamides ou N,N-dichloro sulfonamides, pour l'élimination de composés organiques volatils d'un espace au-dessus d'une plaie, dans lesquelles le groupement tel que lié aux particules de polymère ou fibres de polymère est d'une structure choisie parmi les suivantes :

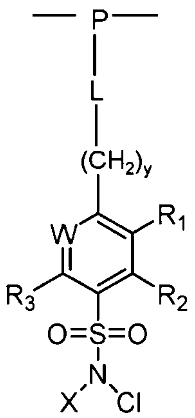
,
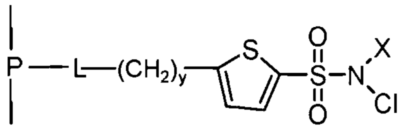
,

$P\text{-}L\text{-}(CH_2)_y\text{-}SO_2\text{-}NXCl$ ou $P\text{-}L\text{-}(CH_2)_y\text{-}NXCl$

où :

P- est un polymère ;
L est un lieur ;
y est un nombre entier supérieur à zéro ;
W est N ou -CH;
$R_1$, $R_2$ et $R_3$ sont indépendamment choisis parmi -H-, -alkyle ou -halogène ;
X est Na, H ou Cl.

**2.** Particules de polymère ou fibres de polymère selon la revendication 1, dans lesquelles

$L\text{-}(CH_2)_y$ est $\text{-}NH\text{-}(CH_2)_y$ ; et/ou
y est compris entre 1 et 20, préférentiellement entre 1 et 12 ou entre 1 et 6 et/ou
$R_1$ est choisi parmi -H-, $\text{-}CH_3\text{-}$ ou -Cl- ; et/ou
un halogène est F, Cl ou Br et/ou :
$R_2$ et $R_3$ sont -H-.

**3.** Particules de polymère ou fibres de polymère selon la revendication 1 ou la revendication 2, dans lesquelles le polymère est à base d'acide polyacrylique, de polyalcool de vinyle ou de polyalcool de vinyle avec groupements acétyle partiellement hydrolysé, éventuellement d'un réseau polymère réticulé d'acide polyacrylique, de polyalcool de vinyle ou de polyalcool de vinyle avec groupements acétyle partiellement hydrolysé, ou de tout copolymère ou mélange de ceux-ci, préférentiellement dans lesquelles le polymère est à base d'acide polyacrylique.

**4.** Particules de polymère ou fibres de polymère selon l'une quelconque des revendications précédentes, dans lesquelles au moins 0,1 % des groupes alcool ou acide carboxylique présents à l'origine dans le polymère sont fonctionnalisés avec une unité N-chloro ou N,N-dichloro, préférentiellement au moins 0,5 %.

**5.** Particules de polymère ou fibres de polymère selon l'une quelconque des revendications précédentes, dans lesquelles la quantité de chlore actif telle que déterminée par la méthode fournie dans la description est comprise entre 0,1 % et 20 % (p/p), éventuellement entre 0,3 % et 15 %.

**6.** Particules de polymère ou fibres de polymère selon l'une quelconque des revendications précédentes, dans lesquelles le diamètre moyen de particules D50 des particules de polymère est compris entre 80 µm et 600 µm,

éventuellement entre 150 μm et 400 μm ; ou dans lesquelles le diamètre moyen des fibres est compris entre 1 μm et 300 μm, éventuellement entre 5 μm et 100 μm.

**7.** Pansement comprenant les particules polymères ou les fibres polymères selon l'une quelconque des revendications précédentes.

**8.** Pansement selon la revendication 7, comprenant au moins 5 % en poids desdites particules de polymère ou fibres de polymère, par rapport au poids total du pansement, éventuellement au moins 10 % en poids et/ou dans lequel les particules de polymère ou fibres de polymère de l'une quelconque des revendications précédentes ont un grammage d'au moins 15 g/$m^2$, éventuellement au moins 30 g/$m^2$ du pansement global.

**9.** Pansement selon la revendication 7, comprenant en outre au moins un élément parmi les suivants :

(a) une couche de revêtement arrière ;
(b) au moins une couche absorbante ;
(c) une couche de contact avec la plaie, ladite couche de contact avec la plaie comprenant éventuellement un gel de silicone.

**10.** Pansement selon la revendication 8 ou la revendication 9, dans lequel les particules de polymère ou les fibres de polymère sont fournies en tant que partie de la couche absorbante, éventuellement dans lequel la couche absorbante comprend des particules superabsorbantes ou des fibres superabsorbantes non fonctionnalisées avec des groupes N-chloro ou N,N-dichloro.

**11.** Pansement selon l'une quelconque des revendications 7 à 10, dans lequel le pansement comprend également des particules superabsorbantes ou des fibres superabsorbantes non fonctionnalisées avec des N-chloro ou N,N-dichloro sulfonamides, dans lequel les particules de polymère ou les fibres de polymère fonctionnalisées avec des N-chloro ou N,N-dichloro sulfonamides sont fournies séparément des particules superabsorbantes ou des fibres superabsorbantes non fonctionnalisées avec des N-chloro ou N,N-dichloro sulfonamides, en particulier dans lequel les particules de polymère ou les fibres de polymère fonctionnalisées avec des N-chloro ou N,N-dichloro sulfonamides sont fournies dans une couche plus éloignée de la zone du pansement qui est destinée à être mise en contact avec la plaie qu'une couche qui comprend les particules superabsorbantes ou les fibres superabsorbantes non fonctionnalisées avec des groupes N-chloro ou N,N-dichloro.

**12.** Procédé de contrôle de l'odeur au-dessus d'une plaie, ledit procédé comprenant au moins les étapes suivantes :

(a) fourniture de particules de polymère ou de fibres de polymère selon l'une quelconque des revendications 1 à 6 dans un pansement ;
(b) mise en contact d'au moins une fraction desdites particules de polymère ou fibres de polymère avec au moins un composé organique volatil qui est exsudé d'une plaie et/ou de la peau humaine à proximité d'une plaie ;
(c) mise en réaction chimique dudit composé organique volatil avec le groupe **N-**chloro ou N,N-dichloro tel que lié de manière covalente auxdites particules de polymère ou fibres de polymère, réduisant ainsi au moins partiellement l'odeur émanant d'une plaie ou de la zone autour d'une plaie.

**13.** Procédé selon la revendication 12, dans lequel ledit au moins un composé organique volatil est choisi parmi des composés hétéroaryliques, aryliques, des sulfures, des disulfures, des trisulfures, des cétones, des alcools, des aldéhydes, des amines ou des acides et esters carboxyliques.

**14.** Procédé selon la revendication 12, dans lequel ledit au moins un composé organique volatil est choisi parmi l'indole, le 3-méthylindole, la pyrimidine, l'acétophénone, l'acide isovalérique, la 2,5-diméthylpyrazine, le pyrrole, le p-crésol, la 2-aminoacétophénone, la 6-méthylhept-5-ène-2-one, la cadavérine.

**15.** Pansement ou procédé selon l'une quelconque des revendications 1 à 14, dans lequel la plaie est choisie parmi des plaies chroniques et/ou des plaies infectées.

**Figure 1, Part 1)**

1) N-chloro or N,N-dichlorosulfonamide modified polymer

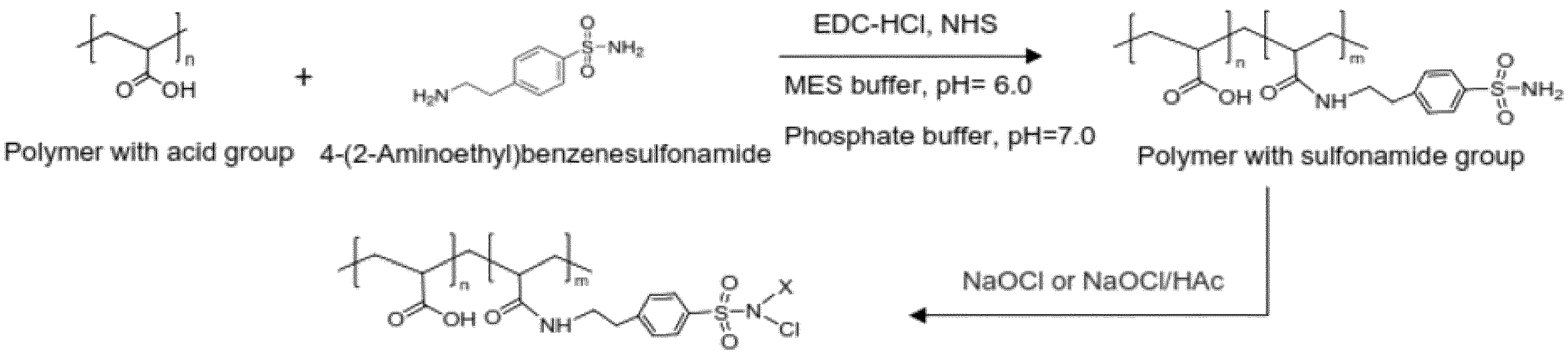


Polymer with acid group + 4-(2-Aminoethyl)benzenesulfonamide

Polymer with sulfonamide group

Polymer with N-chlorosulfonamide group

X = Na, H or Cl

**Figure 1, Part 2)**

2) N-chloro or N,N-dichloroamine modified polymer

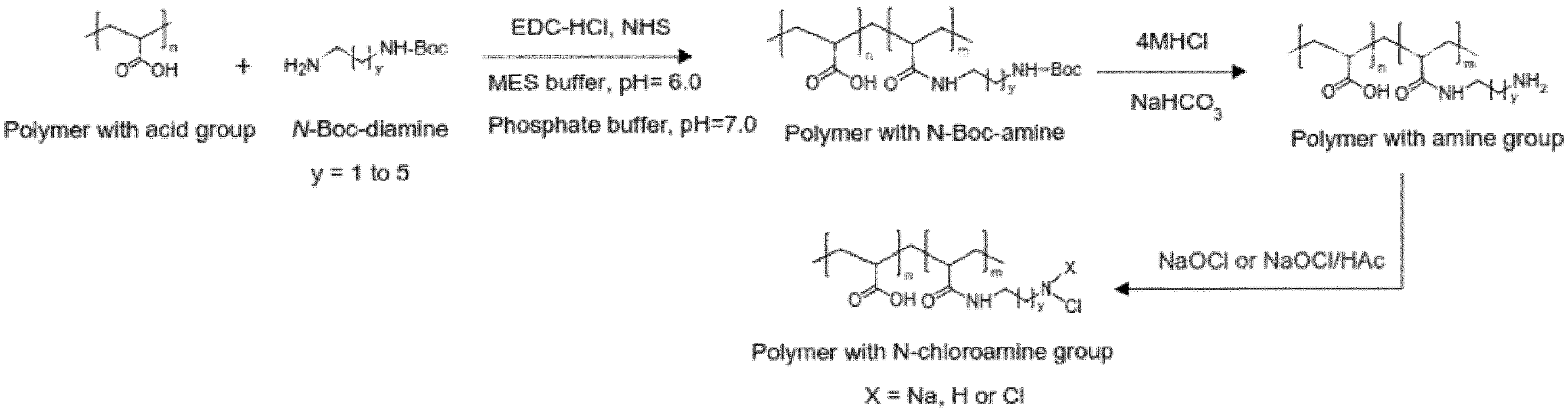


Polymer with acid group

*N*-Boc-diamine

y = 1 to 5

Polymer with N-Boc-amine

Polymer with amine group

Polymer with N-chloroamine group

X = Na, H or Cl

Figure 2, A

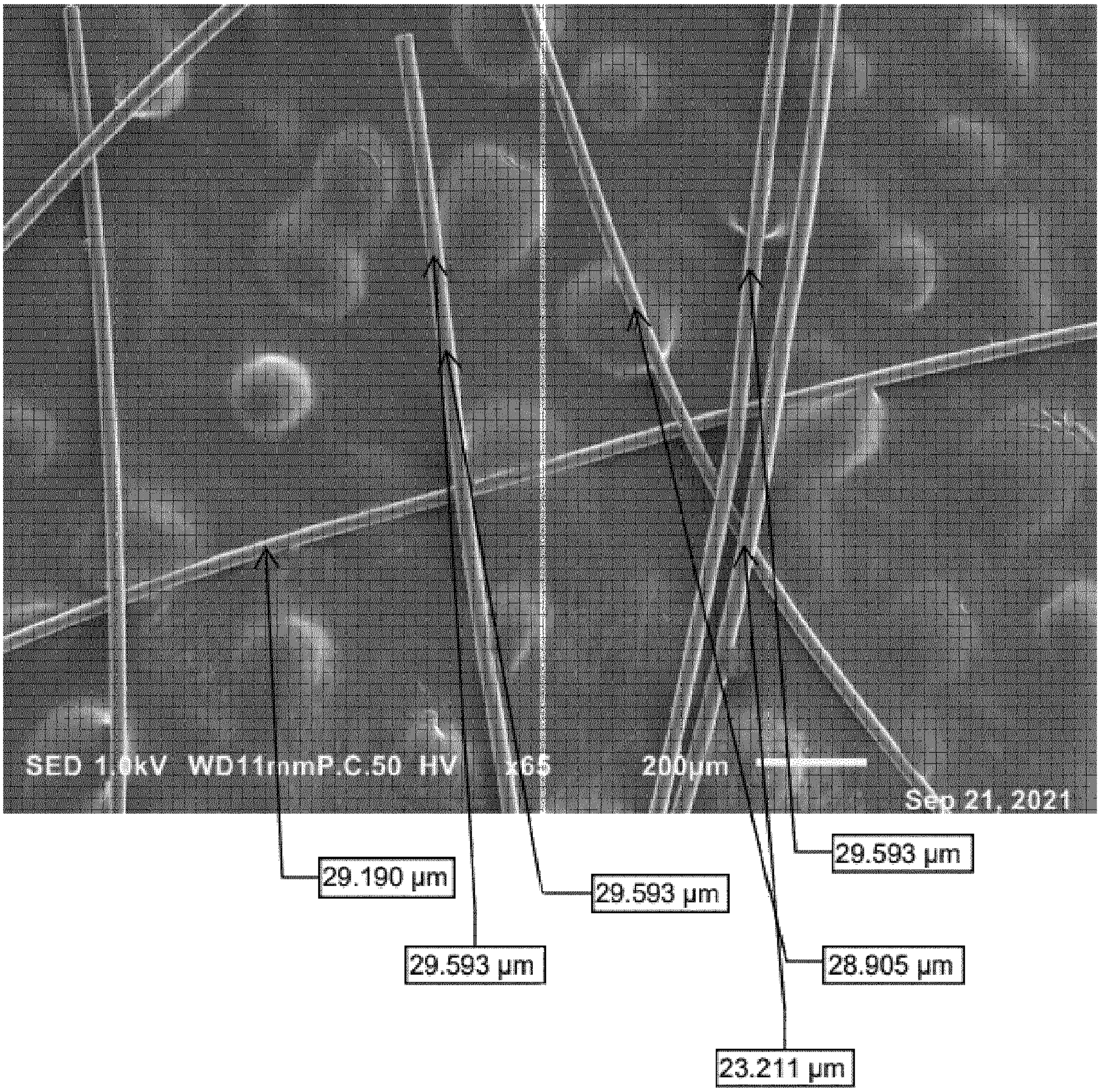
SED 1.0kV WD11mmP.C.50 HV x65 200μm
Sep 21, 2021
29.190 μm
29.593 μm
29.593 μm
29.593 μm
28.905 μm
23.211 μm

Figure 2, B

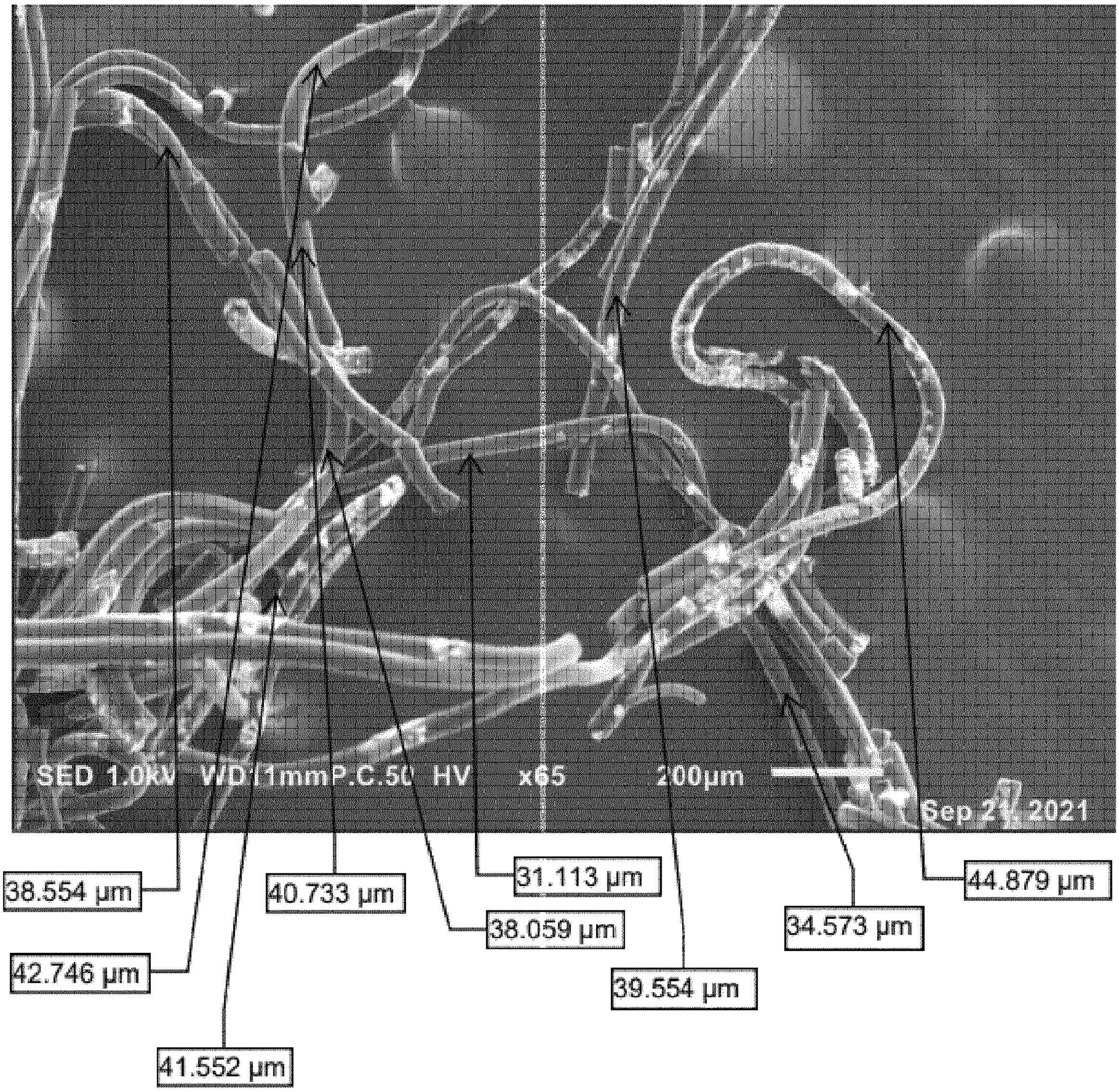
SED 1.0kV WD11mmP.C.50 HV x65 200μm
Sep 21, 2021
38.554 μm
42.746 μm
41.552 μm
40.733 μm
38.059 μm
31.113 μm
39.554 μm
34.573 μm
44.879 μm

Figure 2, C

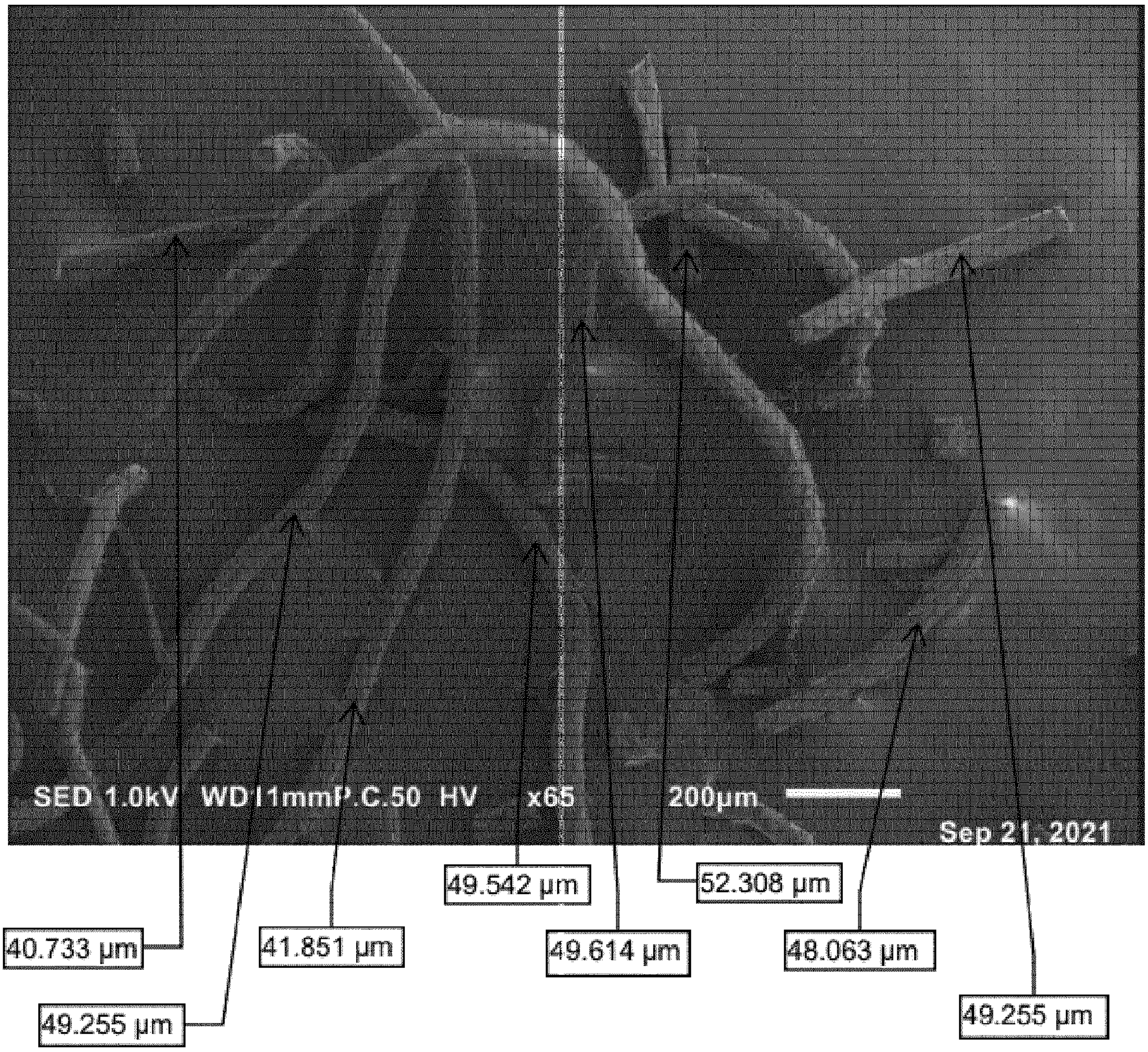
SED 1.0kV WD11mmP.C.50 HV x65 200μm
Sep 21, 2021
49.542 μm
52.308 μm
40.733 μm
41.851 μm
49.614 μm
48.063 μm
49.255 μm
49.255 μm

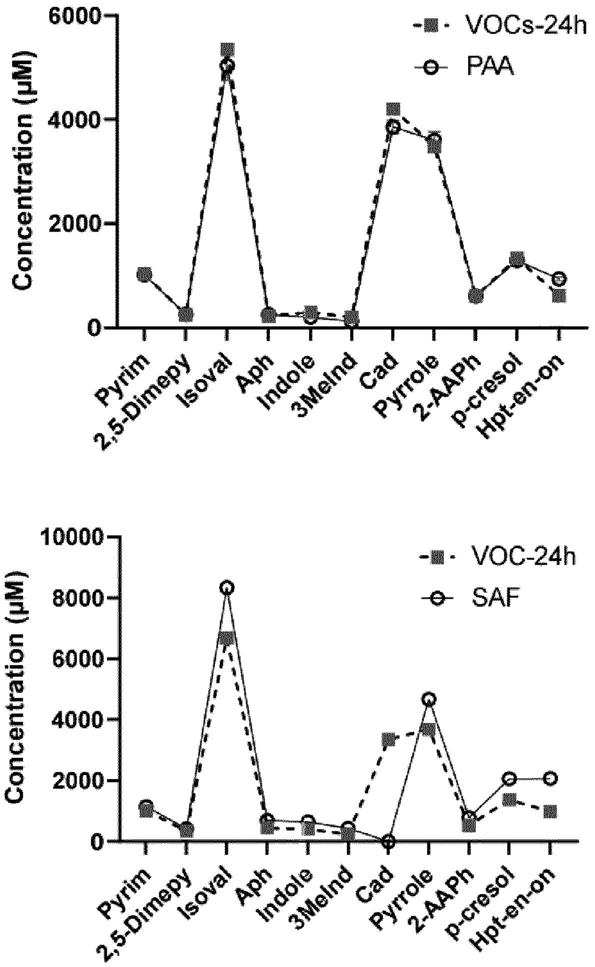

Concentration (µM)
6000
4000
2000
0
VOCs-24h
PAA
Pyrim
2,5-Dimepy
Isoval
Aph
Indole
3MeInd
Cad
Pyrrole
2-AAPh
p-cresol
Hpt-en-on
Concentration (µM)
10000
8000
6000
4000
2000
0
VOC-24h
SAF
Pyrim
2,5-Dimepy
Isoval
Aph
Indole
3MeInd
Cad
Pyrrole
2-AAPh
p-cresol
Hpt-en-on


Figure 3

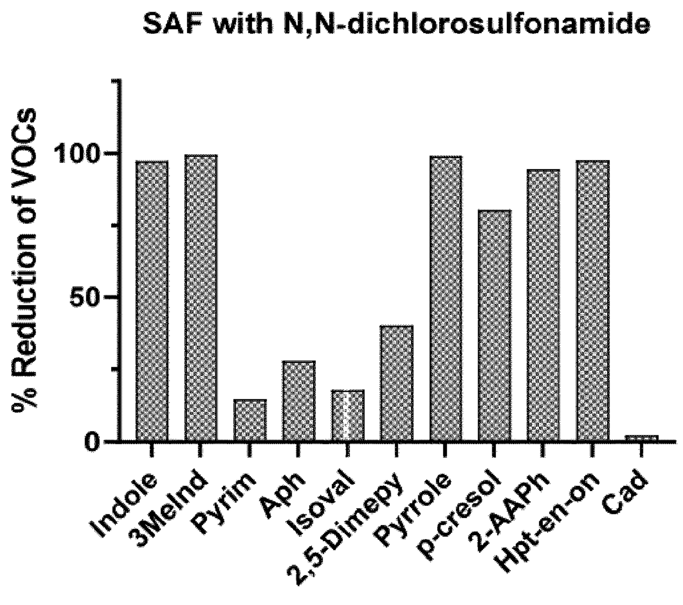
SAF with N,N-dichlorosulfonamide
% Reduction of VOCs
100
50
0
Indole
3MeInd
Pyrim
Aph
Isoval
2,5-Dimepy
Pyrrole
p-cresol
2-AAPh
Hpt-en-on
Cad

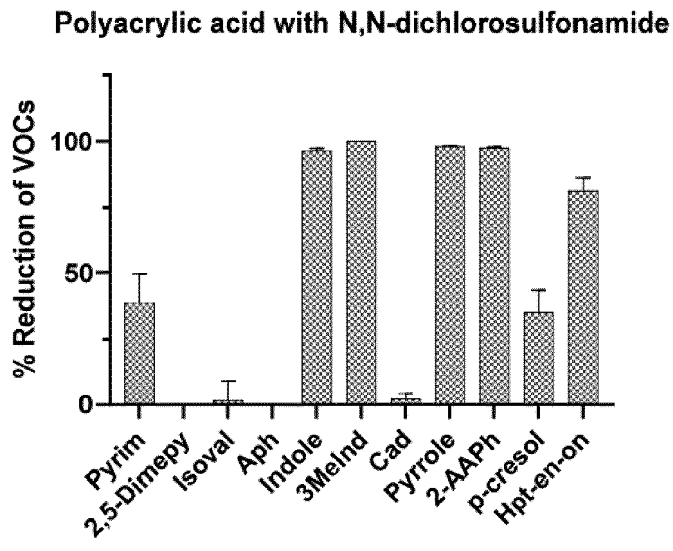
Polyacrylic acid with N,N-dichlorosulfonamide
% Reduction of VOCs
100
50
0
Pyrim
2,5-Dimepy
Isoval
Aph
Indole
3MeInd
Cad
Pyrrole
2-AAPh
p-cresol
Hpt-en-on

Figure 4

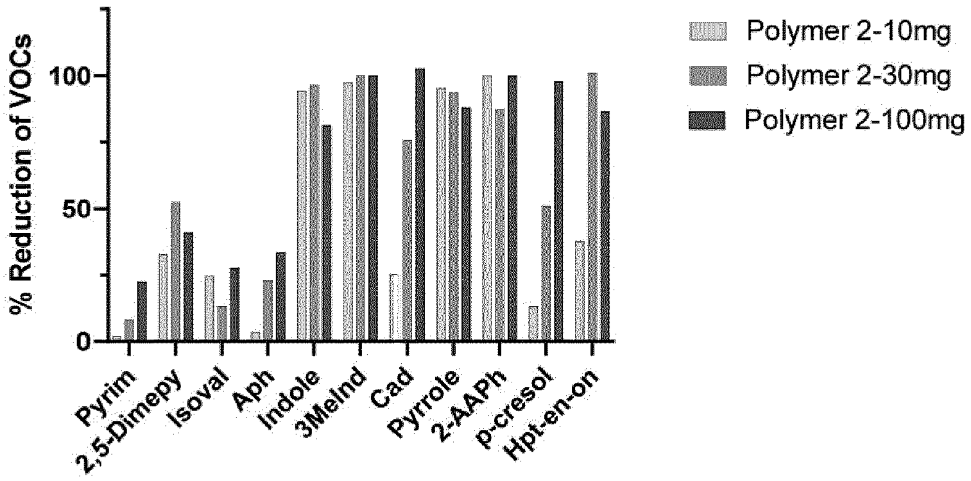


SAF modified with N-Chlorosulfonamide

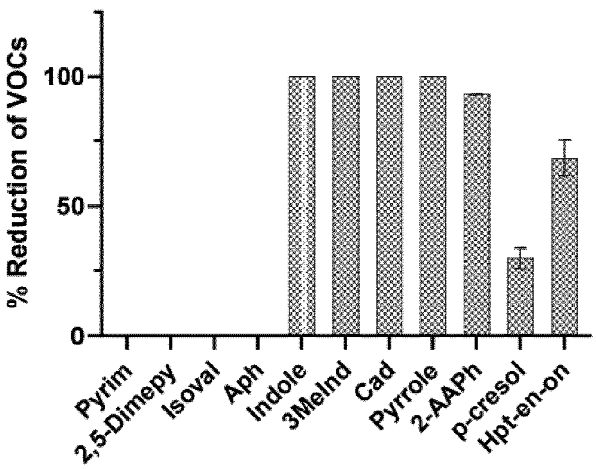


Figure 5

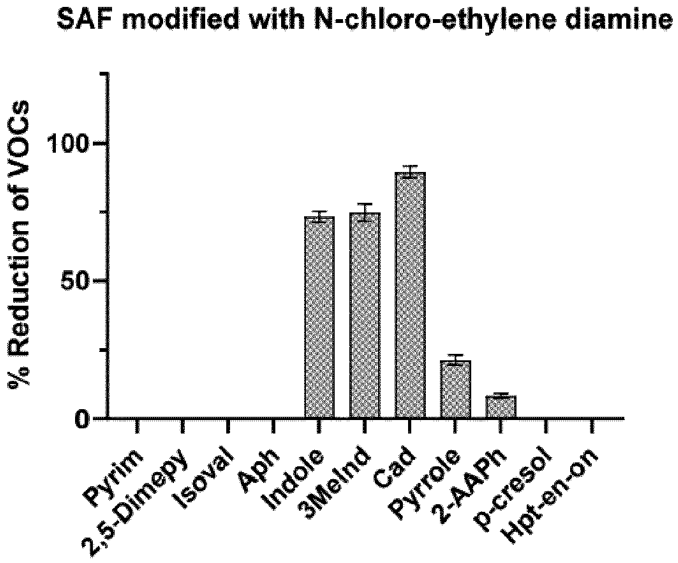
SAF modified with N-chloro-ethylene diamine
% Reduction of VOCs
0
50
100
Pyrim
2,5-Dimepy
Isoval
Aph
Indole
3MeInd
Cad
Pyrrole
2-AAPh
p-cresol
Hpt-en-on

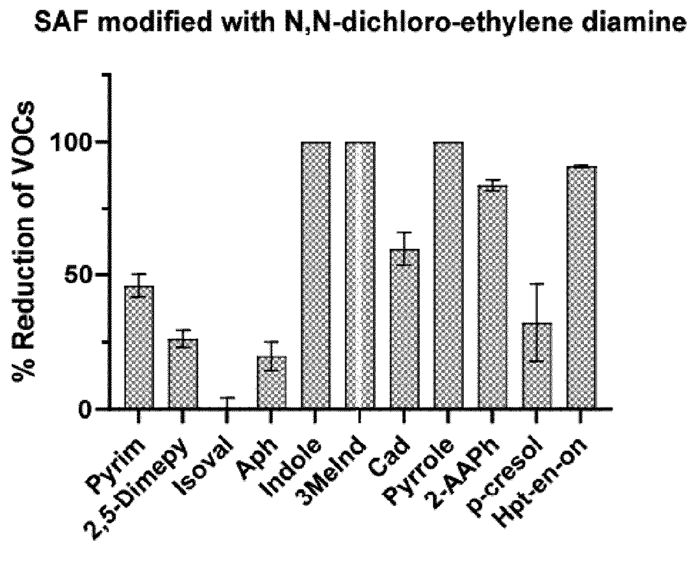
SAF modified with N,N-dichloro-ethylene diamine
% Reduction of VOCs
0
50
100
Pyrim
2,5-Dimepy
Isoval
Aph
Indole
3MeInd
Cad
Pyrrole
2-AAPh
p-cresol
Hpt-en-on

Figure 6

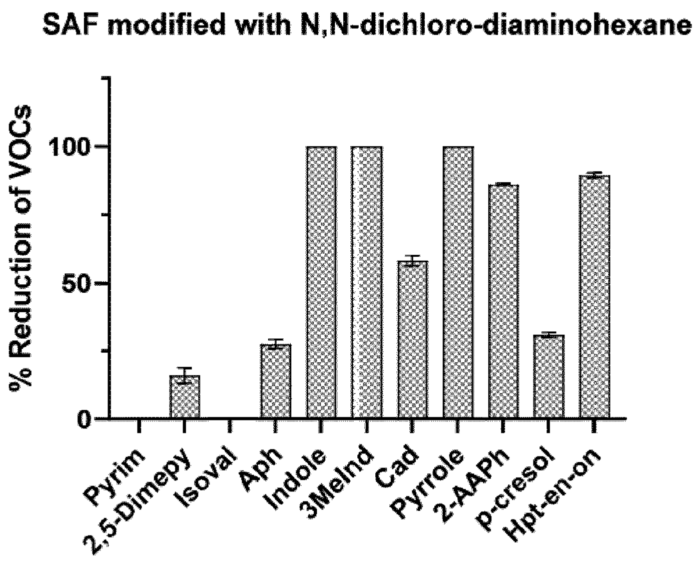
SAF modified with N,N-dichloro-diaminohexane
% Reduction of VOCs
100
50
0
Pyrim
2,5-Dimepy
Isoval
Aph
Indole
3MeInd
Cad
Pyrrole
2-AAPh
p-cresol
Hpt-en-on

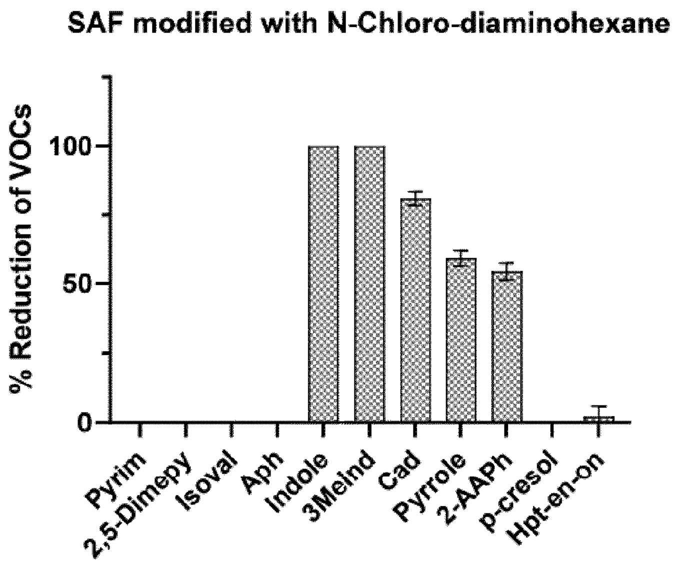
SAF modified with N-Chloro-diaminohexane
% Reduction of VOCs
100
50
0
Pyrim
2,5-Dimepy
Isoval
Aph
Indole
3MeInd
Cad
Pyrrole
2-AAPh
p-cresol
Hpt-en-on

Figure 7

| Materials | % Reduction of VOCs | | | | | | | | | | |
|---|---|---|---|---|---|---|---|---|---|---|---|
| (chlorine content in %) | Pyrim | 2,5-Dimepy | Isoval | Aph | Indole | 3MeInd | Cad | Pyrrole | 2-AAPh | p-cresol | Hpt-en-on |
| Polymer 2 (4%) 100 mg | 14.69 | 40.02 | 17.96 | 27.77 | 97.21 | 99.48 | 2.22 | 99.15 | 94.45 | 80.48 | 97.45 |
| Polymer 2 (12-16%) 10 mg | 2.01 | 32.70 | 24.53 | 3.48 | 94.19 | 97.39 | 25.027 | 95.29 | 100 | 13.15 | 37.50 |
| Polymer 2 (12-16%) 30 mg | 8.13 | 52.59 | 13.17 | 23.02 | 96.38 | 100 | 75.75 | 93.62 | 87.38 | 51.16 | 100 |
| Polymer 2 (12-16%) 100 mg | 22. 42 | 40.84 | 27.65 | 33.43 | 81.19 | 100 | 100 | 87.95 | 100 | 97.88 | 86.53 |

**Figure 8**

| Materials (chlorine content in %) | % Reduction of VOCs | | | | | | | | | | |
|---|---|---|---|---|---|---|---|---|---|---|---|
| | Pyrim | 2,5-Dimepy | Isoval | Aph | Indole | 3MeInd | Cad | Pyrrole | 2-AAPh | p-cresol | Hpt-en-on |
| Polymer 5 (~0.3 - 0.6 %) | 0 | 0 | 0 | 0 | 73.25 | 74.7 | 89.5 | 21.4 | 8.3 | 0 | 0 |
| Polymer 4 (~8%) | 45.9 | 26.1 | 0 | 19.7 | 100 | 100 | 59.7 | 100 | 83.7 | 32.2 | 90.85 |
| Polymer 7 (~1%) | 0 | 0 | 0 | 0 | 100 | 100 | 80.9 | 59.3 | 54.4 | 0 | 0 |
| Polymer 6 (~8%) | 0 | 16.1 | 0 | 27.5 | 100 | 100 | 58.36 | 100 | 86.12 | 30.93 | 89.3 |
| Polymer 3 (~2%) | 0 | 0 | 0 | 0 | 100 | 100 | 100 | 100 | 92.9 | 29.7 | 68.5 |
| Polymer 2 (~14%) | 22.4 | 40.84 | 27.65 | 33.43 | 81.19 | 100 | 100 | 87.95 | 100 | 97.88 | 86.53 |

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8425890 B
- US 8425890 A


### Non-patent literature cited in the description


- **A. AKHMETOVA et al.** A Comprehensive Review of Topical Odor-Controlling Treatment Options for Chronic Wounds. *J Wound Ostomy Continence Nurs.*, 2016, vol. 43 (6), 598-609
- *Pure Appl. Chem.*, 2004, vol. 76 (4), 889-906